# EUROPÄISCHE PATENTSCHRIFT

(11) **EP 0 521 333 B1**
(45) Veröffentlichungstag und Bekanntmachung des Hinweises auf die Patenterteilung: **27.09.1995**
(21) Anmeldenummer: 92110118.4
(22) Anmeldetag: 16.06.1992
(51) Int. Cl.: C04B 28/00, C04B 28/14, A61L 15/08, A61F 13/04, E04B 2/00

(54) **Verfahren zur Herstellung von hydraulische Bindemittel enthaltenden Versteifungsmaterialien, insbesondere Gipsbinden**
Method for making stiffening materials containing a hydraulic binder, especially plaster bandages
Procédé de fabrication de matériaux durcissables contenant un liant hydraulique, en particulier de bandes plâtrées

(30) Priorität: 29.06.1991 DE 4121626
(43) Veröffentlichungstag der Anmeldung: 07.01.1993
(73) Patentinhaber: BAYER AG, 51368 Leverkusen (DE)
(72) Erfinder: von Bonin, Wulf, Dr., W-5068 Odenthal (DE)

(56) Entgegenhaltungen:
- AT-B- 281 671
- DE-A- 1 767 434
- DE-A- 3 001 854
- DE-A- 3 706 094
- DE-A- 4 019 310
- DE-A- 4 036 200
- US-A- 3 649 319

## Beschreibung

Gipsbinden sind seit langem bekannt und werden in großem Umfang für orthopädisch-medizinische Zwecke eingesetzt.

Gipsbinden werden bisher üblicherweise hergestellt, indem man dehydratisierten Gips mit einem in Methylenchlorid gelösten Bindemittel, z.B. einem Bindemittel auf der Basis modifizierter Cellulose, anteigt und diese Paste auf Bindenmaterial, z.B. Baumwollgewebe, aufrakelt. Dann wird das Methylenchlorid durch Verdampfung entfernt, wodurch der Gips durch das Bindemittel an das Bindenmaterial gebunden wird. Nun kann das Gips enthaltende Bindenmaterial geschnitten und gerollt werden, ohne daß der Gips abfällt. Das in Streifen geschnittene, Gips enthaltende Bindenmaterial wird dann auf perforierte, rohrartige Wickelhülsen relativ locker aufgerollt. Die so entstandene Gipsbinde wird aktiviert, indem man sie in Wasser eintaucht. Hierbei bildet der hydrophil gebundene Gips einen Gipsleim, der sich nach der Applikation verfestigt und einen sogenannten Gipsverband ergibt.

Dieses Herstellungsverfahren für Gipsbinden weist den Nachteil auf, daß Lösungsmittel verwendet werden müssen, z.B. Methylenchlorid, die aus heutiger Sicht allenfalls in geringen Mengen tolerierbar sind und deren Entfernung auch aus der Abluft sehr aufwendig und teuer ist. Ähnlich wird bei der Herstellung von für technische Anwendungen geeigneten Versteifungsmaterialien verfahren.

Gemäß einer eigenen, älteren, nicht vorveröffentlichten Patentanmeldung werden hydraulische Bindemittel enthaltende Versteifungsmaterialien hergestellt, indem man ein hydraulisches Bindemittel in Pulverform mit einem reaktiven Bindemittel vermischt, diese Mischung auf ein flächiges Material aufbringt, das so beschichtete flächige Material zu Rollen aufwickelt und vor, während und/oder nach dem Aufwickeln die Abbindereaktion des reaktiven Bindemittels ablaufen läßt.

Aus DE 3 706 094 A1 ist eine aus gebranntem Gips, Blähtonkugeln und Textil- oder Kunststoffgewebe gefertigte Gipsbinde bekannt, die sandwichartig in fünf Schichten aufgebaut ist, wobei die innerste Schicht aus Blähtonkugeln, die beiden mittleren Schichten aus Textil- oder Kunststoffgewebe und die beiden Außenschichten aus aufgepreßtem gebrannten Gips bestehen. Das Kennzeichen der dort beschriebenen Gipsbinden ist die gegenseitige Anordnung der Blähtonkugeln und die Art und Weise wie das Gewebe und der gebrannte Gips im Verhältnis zu den Blähtonkugeln angeordnet sind. Nachteilig bei solchen Gipsbinden ist deren inhomogene Gipsschicht.

Es wurde nun gefunden, daß man verbesserte hydraulische Bindemittel enthaltende Versteifungsmaterialien erhält, wenn man in Abwesenheit von Blähtonkugeln
- ein hydraulisches Bindemittel, gegebenenfalls im Gemisch mit anderen Bindemitteln, in Pulverform auf ein flächiges Material aufbringt,
- das hydraulische Bindemittel, gegebenenfalls in Form von Streifen oder Mustern, im Kontakt mit dem flächigen Material verdichtet und
- das so mit verdichtetem hydraulischem Bindemittel versehene flächige Material zu Rollen aufwickelt.

Das wesentliche Merkmal der vorliegenden Erfindung ist das Verdichten des hydraulischen Bindemittels im Kontakt mit dem flächigen Material. Die Mitverwendung von zusätzlichen, vorzugsweise reaktiven, aber auch nichtreaktiven Bindemitteln ist vorteilhaft. Vorzugsweise führt man die erfindungsgemäße Verdichtung so durch, daß das hydraulische Bindemittel dabei seinen pulverigen und rieselfähigen Charakter verliert. Beispielsweise kann man so verfahren, daß man ein mit hydraulischem Bindemittel in einer Menge von 200 bis 800 g/m² beaufschlagtes flächiges Material durch einen Walzenspalt führt, der eine Weite von 0,02 bis 0,3 mm, vorzugsweise 0,05 bis 0,1 mm aufweist. Die Verdichtung ist dann besonders gut, wenn das hydraulische Bindemittel danach homogenkompakt oder opak-transparent aussieht.

Man kann die Verdichtung beispielsweise auch mit Druckstempeln oder Pressen durchführen.

Eine vorteilhafte Ausführungsform der vorliegenden Erfindung ist dadurch gekennzeichnet, daß man die Verdichtung des hydraulischen Bindemittels auf einer oder zwischen zwei oder mehr Lagen flächigen Materials vornimmt. Letzteres führt zu einer sandwichartigen Struktur des Versteifungsmaterials, z.B. der Gipsbinde. Man kann auch eine Sandwichstruktur mit dem Aufbau verdichtetes hydrauliches Bindemittel/flächiges Trägermaterial/verdichtetes hydrauliches Bindemittel realisieren.

Die Oberfläche der verdichteten Bindemittelschicht kann gegebenenfalls mit Prägemustern versehen werden, z.B. um die Oberfläche zu vergrößern, aus Dekorationsgründen oder zu Kennzeichnungszwecken.

Bei einer weiteren vorteilhaften Ausführungsform der vorliegenden Erfindung befindet sich das hydraulische Bindemittel in Form von einzelnen Querstreifen einseitig oder beidseitig auf dem flächigen Material, so daß eine Strickleiterstruktur entsteht.

Eine sandwichartige Struktur verleiht dem Versteifungsmaterial vor dem Abbinden des hydraulischen Bindemittels besonders gute Verarbeitungseigenschaften. Das hydraulische Bindemittel hat auch dann praktisch keine Tendenz mehr beim Aufrollen oder sonstigen mechanischen Einflüssen wegzurieseln, wenn es wenig oder keine reaktive Bindemittel enthält. Die Mitverwendung von reaktiven Bindemitteln ist trotzdem bevorzugt, da dann beim Tränken mit Wasser zur Abbindung des hydraulischen Bindemittels weniger bis keine Tendenzen zum Schmieren und Ablaufen auftreten.

Deshalb ist neben der Ausführungsform mit einer Lage des flächigen Materials eine besonders günstige Ausführungsform der vorliegenden Erfindung dadurch gekennzeichnet, daß man zur Herstellung von medizinisch anwendbaren Gipsbinden
- ein hydraulisches Bindemittel auf Gipsbasis mit einem reaktiven Bindemittel vermischt,
- diese Mischung auf ein flächiges Material aufbringt,
- die so erhaltene Beschichtung mit einem weiteren flächigen Material abdeckt, (dieser Schritt entfällt bei der Herstellung von Gipsbinden mit nur einer Lage flächigem Material)
- das Bindemittelgemisch bis zum Verlust seiner Rieselfähigkeit verdichtet,
- das so mit einer verdichteten Bindemittelschicht versehene Produkt locker zu Rollen aufwickelt und
- vor, während und/oder nach dem Aufwickeln die Abbindereaktion des reaktiven Bindemittels ablaufen läßt.

Als hydraulische Bindemittel kommen insbesondere die Gipssorten in Frage, die für die medizinische Gipsbindenherstellung üblich sind. Vorzugsweise handelt es sich dabei um teilhydratisierte Gipse, z.B. um sogenannte α- und/oder β-Stuckgipse, deren Abbindezeit weniger als 10 Minuten betragen kann.

Es kommen aber allein oder im Gemisch auch andere hydraulische Bindemittel in Frage, insbesondere auch zur Herstellung von Versteifungsmaterialien für technische Einsatzzwecke. Als Beispiele seien genannt: andere Gipsmodifikationen als Stuckgips, wie α-Gips, β-Gips und Anhydrit, sowie Portlandzement, Tonerdeschmelzzemente, Aluminiumsilikat-Schnellzemente, Sorellzemente, Zinkoxidzemente, Puzzolanzemente und sonstige mit Wasser unter Erhärtung reagierende Mineralpulver mit Zementcharakter. Es kann sich auch um organische oder gemischt mineralisch-organische Pulver oder Zubereitungen handeln, die mit Wasser unter Aushärtung reagieren.

Bei den für die Erfindung einzusetzenden hydraulischen Bindemitteln in Pulverform kann es sich auch um Gemische aus zwei oder mehreren Komponenten handeln. Dem hydraulischen Bindemittel können gegebenenfalls übliche Beschleuniger, z.B. lösliche Sulfate, oder verzögernde oder rheologische Stellmittel, z.B. Proteine oder Cellulose-Derivate, beigefügt sein.

Gegebenenfalls kann das hydraulische Bindemittel im Gemisch mit reaktiven Bindemitteln zum Einsatz gelangen. Als reaktive Bindemittel kommen die verschiedensten als solche bekannten reaktiven Bindemittel oder Bindemittelgemische in Frage, die vorzugsweise in flüssiger Form als Ein- oder Mehrkomponentensysteme auf das hydraulische Bindemittel aufgebracht werden können und dann durch Reaktion der Gemisch-Komponenten und/ oder durch den Einfluß von Wärme, Luft, Feuchte, Belichtung und/oder durch chemische Reaktion mit einem gegebenenfalls zusätzlich einzubringenden Reaktionspartner und oder mit einem Katalysator, Radikalbildner oder sonstigem Starter zu einer Viskositätserhöhung, z.B. infolge einer Vernetzung oder sonstigen Molekulargewichtserhöhung, gebracht werden können.

Die Viskositätserhöhung kann dabei, zumindest teilweise, auf dem hydraulischen Bindemittel und/oder im anschließend gebildeten Wickel erfolgen oder in diesem zu Ende gebracht werden.

Beispiele für reaktive Bindemittel sind: Ohne Zuhilfenahme von organischen Lösungsmitteln applizierbare Silikate vom Wasserglastyp, Gipse oder Zemente bindende Salze, Epoxide, Polyepoxide, Epoxid-Härter-Kombinationen, unter dem Einfluß von Luft, Ionen- oder Radikalbildnern polymerisierende Doppelbindungssysteme, z.B. mono-, di- oder polyolefinische Monomere, etwa vom Cyanacrylat-, Acrylat-, Vinylester-, Allylester- oder Allylethertyp, vom Typ der Silane, Siloxane oder Silikone (vgl. auch DE-OS 23 57 931), vom Typ der Alkydharze und reaktiven Alkydharzverdünner, vom Typ der Cyanatharze, Phenolharze, Formaldehydharze, der Methylolverbindungen, Methylolether, vom Typ der (Poly-)Isocyanate (beispielsweise Urethonimin-, Oxadiazintrion-, Iminoisoyanurat-, Uretdion-, Isocyanurat-, Uretdiimin-, Biuret- und/oder Harnstoff-Gruppen aufweisende bi- oder höherfunktionelle Polyisocyanate auf der Basis aliphatischer Polyisocyanate, z.B. auf der Basis von Hexamethylendiisocyanat, sowie die später im Zusammenhang mit Polyol-Polyisocyanat-Kombinationen genannten Polyisocyanate und Isocyanatprepolymere), die beispielsweise unter Bildung von Polyurethanen, Polyharnstoffen, Polycarbodiimiden und/oder Polyisocyanuraten beispielsweise mit Wasser oder Polyolen zur Reaktion gebracht werden können, gegebenenfalls unter Beteiligung der im Wickel enthaltenen Feuchtigkeit.

Es kommen auch Polycarbonsäuren, Alginate, Aluminate, Cellulose- und Stärkeverbindungen in Betracht, die z.B. mit Calciumionen oder Aluminiumionen aus dem hydraulischen Bindemittel, mit dem sie in Kontakt kommen, einer Fällung oder Härtung unterliegen können.

Als reaktive Bindemittel gelangen vorzugsweise Kombinationen von einem oder mehreren Polyolen und einem oder mehreren Polyisocyanaten zum Einsatz. Kombinationen aus Polyolen und Polyisocyanaten können gegebenenfalls noch weitere Komponenten enthalten, vorzugsweise solche, die die Reaktion zwischen dem Polyol und dem Polyisocyanat verzögern oder beschleunigen. Beschleunigende Zusätze sind bevorzugt. Solche weitere Komponenten, z.B. Katalysatoren, können gegebenenfalls in Mengen von 0,05 bis 3 Gew.-% (bezogen auf das Gemisch aus Polyol und Polyisocyanat) eingesetzt werden.

Als beschleunigende Zusätze kommen z.B. aminische oder metallorganische Verbindungen oder auch sonstige, in der Polyurethanchemie als katalytisch wirksam bekannte Verbindungen in Frage. Diese können auch, z.B. in Form von Amingruppen, in dem Polyol integriert sein.

Die Komponenten des reaktiven Bindemittels können in gemischter Form, getrennt und gleichzeitig oder vorzugsweise nacheinander dem pulverförmigen hydraulischen Bindemittel zugemischt werden.

Dem hydraulischen Bindemittel, vorzugsweise dem Gips, kann das reaktive Bindemittel beispielsweise in Mengen von 0 bis 50 Gew.-%, vorzugsweise 0,5 bis 10 Gew.-%, insbesondere 2 bis 6 Gew.-%, zugemischt werden. Es ist vorteilhaft darauf zu achten, daß das Gemisch aus hydraulischem Bindemittel und reaktivem Bindemittel noch pulverförmigen, streubaren Charakter behält.

Es ist weiterhin von Vorteil, aber nicht zwingend notwendig, ein frisch hergestelltes Gemisch aus hydraulischem und reaktivem Bindemittel unmittelbar oder innerhalb weniger Stunden nach der Herstellung auf das flächige Material aufzubringen. Vor dem Aufbringen auf das flächige Material können dem Gemisch aus hydraulischem und reaktivem Bindemittel oder einer einzelnen Komponenten davon gegebenenfalls weitere Zusatzstoffe, wie Netzmittel, Tenside, Fließhilfsmittel, Farbpigmente und/oder Biocide, hinzugefügt werden.

Auf 1 m² des flächigen Materials können z.B. 100 bis 1000 g, vorzugsweise 150 bis 800 g, insbesondere 200 bis 600 g hydraulische Bindemittel oder Gemische aus hydraulischen, reaktiven und/oder nicht-reaktiven Bindemitteln aufgebracht werden.

Bei den bevorzugten reaktiven Bindemitteln, den Kombinationen aus Polyolen und Polyisocyanaten, kommen als Polyole vorzugsweise die in der Polyurethanchemie technisch zur Verwendung kommenden linearen und verzweigten, vorzugsweise bei Raumtemperatur flüssigen Polyesterpolyole und insbesondere Polyetherpolyole in Betracht. Von besonderem Interesse sind trifunktionelle und höherfunktionelle Typen, die durch Anlagerung von Ethylenoxid und/oder Propylenoxid an tri- und höherfunktionelle Starter, z.B. an Trimethylolpropan, Glycerin, Pentaerythrit, Sorbit, Zucker oder Zuckergemische, Ammoniak, Triethanolamin, Ethylendiamin, Polyethylenpolyamin, Polypropylenpolyamin, Ethanolamin und/oder Diethanolamin erhalten werden können und OH-Zahlen über 5, vorzugsweise zwischen 30 und 400, insbesondere zwischen 150 und 300, aufweisen.

Als Polyisocyanate kommen vorzugsweise ebenfalls in der Polyurethanchemie technisch zur Anwendung kommende aliphatische, araliphatische, heterocyclische und aromatische Polyisocyanate in Betracht. Vorzugsweise werden solche Polyisocyanate verwendet, deren Dampfdruck im Bereich 10 bis 50°C sehr niedrig ist. Dann ist bei deren Handhabung keine Gefährdung zu befürchten. Bei derartigen Polyisocyanaten handelt es sich vorzugsweise um bei Raumtemperatur flüssige, vorzugsweise aliphatische Polyisocyanate wie polymerisiertes, trimerisiertes, biuretisiertes, allophanatisiertes bzw. mit geringen Mengen Wasser oder Kohlendioxid umgesetztes Hexamethylendiisocyanat, Isophorondiisocyanat oder solche Typen von gegebenenfalls aromatischen Mehrkernpolyisocyanaten, wie sie auf den Wegen der Phosgenierung von Anilin-Formaldehyd-Kondensaten und deren hydrierten Formen technisch zugänglich sind. Es kommen auch andere flüssige Polyisocyanate in Betracht, auch aliphatisch-aromatische Mischtypen, z.B. solche auf Basis Isophorondiisocyanat oder Toluylendiisocyanat oder sogenannte Isocyanat-Prepolymere, d.h. Isocyanatgruppen enthaltende, vorzugsweise flüssige, oligomere Umsetzungsprodukte von Polyolen mit Polyisocyanaten.

Es ist von Vorteil, aber nicht zwingend, wenn die Komponenten solcher reaktiven Bindemittelmischungen aus Polyol und Polyisocyanat ineinander löslich sind. Es können auch Polyolgemische und/oder Polyisocyanatgemische zum Einsatz kommen.

Das stöchiometrische Verhältnis von OH- zu NCO-Gruppen in Polyol-Polyisocyanat-Gemischen kann in weiten Grenzen schwanken. Vorzugsweise werden mit Abweichungen von +/-50 Gew.-% stöchiometrische Verhältnisse eingehalten. In speziellen Fällen ist es auch möglich, das Verhältnis der eingesetzten Polyisocyanate und Polyole bis auf jeweils 3 Gew.-% der stöchiometrisch äquivalenten Menge oder auch noch darunter zu reduzieren. Besonders bevorzugt ist der Einsatz von reaktiven Bindemitteln, die 30 bis 90 Gew.-% der in Bezug auf die Polyolkomponente stöchiometrisch erforderlichen Menge an Polyisocyanatkomponenten enthalten.

Diese Verhältnisse gelten auch dann, wenn man die reaktive Kombination flüssiger Bindemittelkomponenten vor, während oder nach dem Eintrag in das pulvrige hydraulische Bindemittel herstellt.

Anstelle von oder zusätzlich zu reaktiven Bindemitteln können auch nicht-reaktive Bindemittel verwendet werden, z.B. ethoxylierte Abietinsäure.

Als flächiges Material, auf das ein hydraulisches Bindemittel oder eine Mischung aus hydraulischen und/oder reaktiven Bindemitteln aufgebracht wird, im folgenden auch vereinfacht als "Bindengewebe" bezeichnet, kommen die verschiedensten flexiblen, vorzugsweise textilen Substrate in Frage. Sie können beispielsweise aus Fäden, Fasern, Drähten oder Folienbändchen bestehen. Vorzugsweise handelt es sich um Vliese, Papiere, Gewirke, Gestricke oder Gewebe oder Mischformen davon.

Vorzugsweise werden aus Baumwolle gefertigte Bindengewebe eingesetzt, wie sie für die konventionelle Gipsbindenfertigung üblich sind. Als textile Substrate kommen aber auch solche in Betracht, die unter Verwendung von z.B. Glasfasern, Kohlenstoffasern, Polyaramidfasern, Metallfasern, Polyolefinfasern, Polyolefin-, Polyamid- und Polyester-Hochmodulfasern, sonstige Polyesterfasern, sonstige Polyacrylnitrilfasern, Seide, sonstige Polyamidfasern und Fasern aus veredelter Cellulose sowie aus Fasergemischen und/oder Fadengemischen hergestellt worden sind. Auch sogenannte Feinfasern, Hohlfasern und Microfasern kommen in Betracht.

Das einlagig oder mehrlagig, vorzugsweise ein- oder zweilagig (sandwichartig) angeordnete flächige Material kann vor oder nach dem Aufbringen des hydraulischen Bindemittels oder bevorzugt vor oder nach dem der Konfektionierung dienenden Aufwickeln in Länge bzw. Breite zugeschnitten werden, z.B. zu Einzelbinden.

Das Aufbringen des pulvrigen Bindemittels, das gegebenenfalls ausreagierte oder nicht ausreagierte reaktive Bindemittel und/oder nicht reaktive Bindemittel enthalten kann, kann flächig oder in Form von Streifen oder sonstigen Mustern im Umkehr- oder, vorzugsweise im Direktverfahren z.B. durch Aufstreuen, Aufblasen, Aufrakeln, auf elektrostatische Weise oder nach beliebigen anderen, keine Lösungsmittel erfordernden Verfahren erfolgen.

Das hydraulische Bindemittel oder Bindemittelgemisch kann auf dem flächigen Material eine homogene Fläche bilden, die gegebenenfalls später mit Mustern versehen werden kann, z.B. mit streifenförmigen Mustern durch einen Prägevorgang. Das Bindemittel kann aber auch in Form von gegebenenfalls verschiedene Schichtdicken aufweisenden Streifen, Punkten, durchbrochenen Flächen oder in Form von Mustern ein- oder beidseitig aufgebracht werden, beispielsweise um das Tränkwasser besser eindringen zu lassen oder um gezielte Versteifungseffekte zu erreichen. Bevorzugt wird das Bindemittel in Form von Wickelachsen-parallelen Streifen oder in Form von Punkten oder Mustern aufgebracht.

Gegebenenfalls kann eine einmal aufgebrachte Schicht des Bindemittels z.B. durch Rütteln, Vibration oder pneumatische Prozesse zur Ausbildung inhomogener Verteilungen und/oder durchlässigerer Bereiche gebracht werden.

Die Herstellung von Gipsbinden mit einer doppelseitigen Leiterstruktur kann auf verschiedene Art leicht erfolgen. Beispielsweise kann man einen nach Art der gewünschten Leiterstruktur gerippten Mitläufer (z.B. vom Typ "Panzerkette") aus Metall oder Kunststoff mit Bindemittel bestreuen, dann eine Lage Bindengewebe auflegen, dieses wieder mit Bindemittel bestreuen und dann den Mitläufer zusammen mit dem aufgelegten und bestreuten Bindengewebe durch ein Walzenpaar laufen lassen. Dabei bewirkt die Leiterstruktur des Mitläufers eine beidseitige Verbindung des Bindemittels auf dem Bindengewebe. Je nach der Festigkeit des Bindengewebes können Preßdrucke von beispielsweise etwa 3 bis über 300 bar Anwendung finden.

Solche doppelseitigen Leiterstrukturen können gegebenenfalls auch mit einem paar Riffelwalzen mit Metall- oder Kunststoffoberfläche hergestellt werden. Man kann dabei das Bindengewebe senkrecht zwischen dem Walzenpaar hindurch führen und beidseitig über die Walzen oder in den Walzenspalt das pulverige Bindemittel dosiert zuführen. So kann kontinuierlich und schnell eine Gipsbinde mit doppelseitiger Leiterstruktur hergestellt werden. Zweckmäßigerweise wird die Einstellung des Walzenspaltes über den Anpreßdruck der Walzen kontinuierlich gesteuert. Die Walzen müssen synchron laufen, d.h. die flächigen Grate der Riffelung und die Spalten zwischen diesen Graten stehen sich jeweils in Passung gegenüber.

Der Lagenverbund beidseitig mit Gips beaufschlagter Binden ist besonders gut.

Es ist im allgemeinen von Vorteil, die für das Aufbringen des Bindemittels eingesetzten Maschinen und Werkzeuge nichthaftend auszurüsten, z.B. durch Überzüge aus Polyolefinen, Silikonen, Perfluorpolyethylenen oder durch die Verwendung von Trennfolien oder durch gute Polierung.

Der an den Beschichtungsprozeß anschließende erfindungswesentliche Verdichtungsvorgang führt, gegebenenfalls in Kombination mit einer oder mehreren auf die Beschichtung aufgelegten flächigen Materialien zu einer verbesserten Trocken-Handhabbarkeit des Versteifungsmaterials, d.h. beim mechanischen Handhaben, z.B. Aufwickeln, des noch nicht hydraulisch abgebundenen Versteifungsmaterials treten praktisch keine Verluste durch Wegrieseln mehr auf. Außerdem tritt beim Eintauchen des aufgewickelten Versteifungsmaterials in Wasser praktisch kein Verlust mehr durch Ablaufen auf. Dies ist auch der Fall, wenn das hydraulische Bindemittel in Form von Wickelachsen-parallelen Streifen oder in sonstigen Mustern angeordnet ist.

Es kann auch mehr als eine Gewebelage unter der Beschichtung plaziert und/oder das Bindemittel-Pulver vor dem Verdichtungsprozeß mit mehr als einer Lage flächigen Material abgedeckt werden. Die einzelnen Lagen flächigen Materials können gleich- oder verschiedenartig sein.

Das Verdichten, z.B. durch Pressen, führt überraschend leicht zu einer Beseitigung der Rieselfähigkeit des pulverigen, gegebenenfalls nicht-reaktive, vorzugsweise reaktive Bindemittel enthaltenden hydraulischen Bindemittels, und man kann es auch an relativ grobmaschigem Bindengewebe fixieren, wie es für medizinische Gipsbinden bisher üblicherweise verwendet wird. Das so fixierte Beschichtungsgemisch bleibt überraschenderweise auch bei Bewegung der Binde, z.B. bei deren Aufrollung und Hand habung auf dem flächigen Material oder zwischen mehreren Lagen flächigen Materials fixiert und rieselt nicht heraus.

Üblicherweise kommen als flächige Materialien Bindengewebe aus Baumwolle nach Art des Verbandmulls zum Einsatz. Diese können z.B. 25 bis 35 große und 25 bis 35 kleine Maschen pro cm², einen leiterartigen rechteckigen Habitus und paarweise in Abständen von 0,5 bis 1,5 und 1,5 bis 2,5 mm laufende Schuß- und Kettfäden aufweisen. Selbstverständlich kommen als flächige Materialien auch andere Gewebe- und Textiltypen in Betracht.

Bei Auflagemengen des pulverigen Bindemittels von z.B. 200 bis 800 g/m² wird das Verdichten vorzugsweise kontinuierlich mit einem Durchlauf durch ein oder mehrere Walzenpaare vorgenommen, wobei deren Walzenachsen vorzugsweise parallel und zweckmäßigerweise horizontal übereinander oder nebeneinander angeordnet sind. Bei Walzen, z.B. solchen aus unprofiliertem, poliertem Stahl oder Edelstahl, können dann z.B. Spaltbreiten von , 0,02 bis 0,3 mm vorzugsweise 0,05 bis 0,1 mm gewählt werden Diese Spaltbreiten können sich aber ändern, wenn die Walzen z.B. mit federnd variablem oder andruckgesteuertem Spalt arbeiten, wenn es sich um profilierte Walzen (z.B. Riffelwalzen oder Musterwalzen) handelt, oder um Walzen mit einer elastisch nachgiebigen Kunststoffbeschichtung, z.B. aus Polycarbonat oder Gummi. Vorzugsweise wird mit einem Walzenpaar und ohne Friktion gearbeitet.

Unter Riffelwalzen versteht man solche Walzenpaare, bei denen eine oder beide Walzen, vorzugsweise nur eine Walze, einen Zahnrad-ähnlichen Querschnitt senkrecht zur Achse aufweist. Solche Walzen können auf der Oberfläche achsparallele, z.B. V-förmige Einschnitte enthalten. Die Verdichtung erfolgt dann in Form eines Streifenmusters. Der zwischen den Verdichtungsstreifen liegende Anteil des Bindemittels ist dann noch pulverig und kann abgesaugt, abgerieselt oder abgerüttelt und erneut zur Beschichtung verwendet werden. Beim Aufwickeln eines mit Riffelwalzen verdichteten Versteifungsmaterials liegen die Streifen der haftenden Beschichtung parallel zur Wickelachse, wodurch sich das Material besonders gut auf- und abwickeln läßt, ohne daß die Beschichtung abspringt.

Bei dieser Ausführungsform der Erfindung können die Verdichtungsstreifen z.B. eine Breite von 1 bis 20 mm, vorzugsweise 2 bis 6 mm aufweisen und die Zwischenräume zwischen den Verdichtungsstreifen z.B. eine Breite von 0,5 bis 10 mm, vorzugsweise 1 bis 5 mm. Diese Breiten können in speziellen Fällen auch unter- oder überschritten werden.

Anstelle des hier näher beschriebenen Verdichtungsmusters in Form achsparalleler Streifen können auch andere Verdichtungsmuster zur Anwendung kommen, z.B. diagonal verlaufende Streifen, wellenförmig verlaufende Streifen oder streifige oder anders angeordnete Folgen von Punkten. Solche Verdichtungspunkte können z.B. Durchmesser von 1 bis 20 mm, vorzugsweise 2 bis 5 mm aufweisen und z.B. wie Kreise, Quadrate, Rechtecke oder Sterne aussehen. Auch hier können die Maße und Figuren in speziellen Fällen anders gewählt werden.

Verdichtungen in Form von Streifen oder sonstigen Mustern kann man praktisch in allen Fällen zwischen Walzen vornehmen, beispielsweise nicht nur mit entsprechenden Riffelwalzen, sondern auch dadurch, daß man zwei glatte Walzen verwendet und zusätzlich zu dem mit pulverförmigem Bindemittel beschichteten flächigen Material flache Plastik- oder Metallteile, z.B. in einer Strickleiter-Anordnung, durch die Walzen laufen läßt. Dann wird im wesentlichen dort verdichtet, wo solche Plastik- oder Metallteile liegen. Beispielsweise kann man auf diese Weise ein Streifenmuster erhalten, wenn man ein strickleiterförmiges Band aus z.B. Plastik (etwa aus üblichen Thermoplasten wie Polyamid, Polycarbonat, Polyurethan, Polyester oder PVC) oder Metall (etwa aus Stahl, Aluminium oder Messing) durch die Walzen mitführt. Ein derartiges strickleiterförmiges Band kann auf und/oder unter, vorzugsweise unter dem beschichteten flächigen Material durch die Walzen geführt werden.

Die Verwendung von polierten (Edel-)Stahl-Walzen wird bevorzugt. Es kommen aber auch z.B. um die flächigen Materialien zu schonen, Gummi- oder Kunststoffwalzen als eine oder beide Walzen eines Walzenstuhls in Frage.

Die Walzen werden vorzugsweise bei Raumtemperatur betrieben. Es kommen auch erniedrigte oder erhöhte Temperaturen in Frage. Im allgemeinen sollte eine Temperatur von 120°C, vorzugsweise 80°C nicht überschritten werden.

Bei einer besonderen Ausführungsform des Verdichtungsvorgangs wird pulvriges Bindemittel oder Bindemittelgemisch unmittelbar nach seiner Herstellung in den sich vor dem Walzenspalt bildenden Zwickel aus ein- oder zwei flächigen Materialien in definierter Menge eingestreut oder eingetragen. Hierzu sind z.B. Schüttelrutschen, streuaggregate und Schlitzdüsen geeignet.

Die Dichte und Durchlässigkeit des zu Rollen aufgewickelten Materials kann durch den Wickeldruck, d.h. durch Variation der Zugkräfte beim Wickelprozeß gesteuert werden. Vorzugsweise wählt man eine lockere Rollenwicklung, wie sie auch bei den konventionell hergestellten Gipsbinden üblich ist und das Eindringen des Wassers zum Abbinden des hydraulischen Bindemittels erleichtert.

Es ist von Vorteil, daß das erfindungsgemäß vorzugsweise zu verwendende Gemisch aus Gips und zusätzlichem reaktivem Bindemittel auch in nicht abgebundenem Zustand nicht mehr zum Stauben neigt. Das verbessert die Raumluftsituation bei der Bindenherstellung in besonderem Maße.

Bei aufgewickelten Rollen (= Wickel), die reaktive Bindemittel enthalten, bindet dieses im Laufe der Zeit ab. Dieser Abbindeprozeß kann auch in der Endverpackung stattfinden. Durch den erfindungsgemäßen Verdichtungsprozeß in Kombination mit einer solchen Abbindung reaktiver Bindemittel wird das Herausrieseln des in den Wickel eingebrachten hydraulischen Bindemittels weitgehend verhindert.

Nach dem erfindungsgemäßen Verdichtungsprozeß in Kombination mit der Abbindung eines vorzugsweise reaktiven Bindemittels liegt das hydraulische Bindemittel in Form eines gebundenen, verdichteten, feinen Kornes in einer kompakten, opaken bis weißlichen Beschichtung vor. Überraschenderweise behindert diese Struktur nicht das Abbinden des hydraulischen Bindemittels mit Wasser, sondern läßt das Wasser beim Eintauchen des Wickels für z.B. 1 bis 60 Sekunden, vorzugsweise 2 bis 10 Sekunden, sehr schnell und gleichmäßig in diesen eindringen. Dabei wird im allgemeinen nur ein geringer Überschuß an Wasser vom Wickel aufgenommen. Weiterhin wird auch bei längeren Tauchzeiten nur wenig hydraulisches Bindemittel mit dem überschüssigen Wasser aus dem Wickel herausgetragen. Dadurch wird gegenüber der konventionellen Herstellung von Gipsbinden eine signifikante Verbesserung der Sauberkeit bei der Handhabung solcher mit Wasser getränkter Binden erreicht. Andererseits wird durch die gebundene Struktur die Verstreichbarkeit und die Modellierbarkeit der erfindungsgemäß hergestellten Versteifungsmaterialien nicht oder nur wenig beeinträchtigt.

Wenn man auf die erfindungsgemäße Weise Gipsbinden herstellt, kann man das Schneiden der Binden, deren Aufwicklung und Verpackung nach konventionellen Methoden der Gipsbindenherstellung vornehmen. Das Schneiden kann z.B. durch Flammen- oder Lasereinwirkung, mit einem Sand- oder Wasserstrahl, mit Ultraschallmessern, Schneidmessern, Sägen oder Trennscheiben erfolgen. Andere Anwendungen erfindungsgemäß hergestellter Versteifungsmaterialien können ebenfalls auf an sich bekannte Weise erfolgen.

Erfindungsgemäß erhältliche Versteifungsmaterialien können z.B. im medizinisch-orthopädischen Bereich angewendet werden. Dabei können erfindungsgemäß hergestellte Versteifungsmaterialien, insbesondere Gipsbinden, auf übliche Weise aktiviert werden, indem man sie flächig oder aufgerollt auf geeignete, z.B. gelochte Hülsen kurzzeitig in Wasser eintaucht. Die Eintauchzeit kann z.B. 1 bis 30 Sekunden bei einer Temperatur von 0 bis 80°C, vorzugsweise 15 bis 30°C betragen.

Das erfindungsgemäße Verfahren hat den Vorteil, daß es staubfrei aber "trocken" ist, d.h. kein Lösungsmittel benötigt wird. Auch wird keine belastete Abluft erzeugt, die nur aufwendig und kostspielig zu reinigen wäre. Das bedeutet gegenüber dem Stand der Technik eine erhebliche Vereinfachung.

### Beispiele

Im folgenden wird das erfindungsgemäße Verfahren beispielhaft erläutert. Teile und Prozente beziehen sich auf das Gewicht, soweit nichts anderes angegeben ist.

In den Beispielen wurden folgende Materialien verwendet:
10 cm breite und 300 cm lange oder endlose Streifen aus Baumwollgewebe, wie es üblicherweise für die Herstellung von Gipsbinden eingesetzt wird (7 Schußfäden, 10 Kettfäden pro cm², Maschengröße 1 x 1 und 1 x 2 mm, das Metergewicht des Gewebestreifens betrug 2,56 g).

Gipspulvergemisch, wie es als sogenanntes Halbhydrat analog Stuckgips für die Gipsbindenfertigung nach konventionellen Verfahren eingesetzt wird.

Polyol A, ein technisches Anlagerungsprodukt von 80 Mol Propylenoxid und 20 Mol Ethylenoxid an Sorbit, mit einer OH-Zahl von 175.

Polyol B, ein technisches Anlagerungsprodukt von gleichen Teilen Ethylenoxid und Propylenoxid an Glycerin, mit einer OH-Zahl von 250.

Polyol C, ein technisches Anlagerungsprodukt von 60 % Ethylenoxid und 40 % Propylenoxid an Sorbit, mit einer OH-Zahl von 8.

Isocyanat A, ein technisches Biuretisierungsprodukt von Hexamethylendiisocyanat mit einem Isocyanatgehalt von 21 %.

Isocyanat B, ein technisches Mehrkernpolyisocyanat aus der Phosgenierung von Anilin-Formaldehyd-Kondensaten, mit einem Isocyanatgehalt von 31 %.

Isocyanat C, ein durch Umsetzung von 2 Mol Hexamethylendiisocyanat mit einem Mol CO₂ zugängliches Oxadiazintrion-diisocyanat mit einem NCO-Gehalt von 23 %.

Auf einem schnell laufenden Schaufelmischer (Lödigemischer) wurden bei Raumtemperatur aus dem Gips und den Polyolen und Polyisocyanaten folgende Bindemittelgemische hergestellt, die alle rieselfähig oder streufähig waren:
Gemisch 1:
   200 Teile Gipspulver wurden vorgelegt, dazugegeben wurde ein Gemisch aus 9,3 Teilen Polyol A und 3,5 Teilen Polyisocyanat A.
Gemisch 2:
   150 Teile Gipspulver wurden vorgelegt, dazugegeben wurden zunächst 6,9 Teile Polyisocyanat A und dann 10 Teile Polyol A.
Gemisch 3:
   150 Teile Gipspulver wurden vorgelegt, dazugegeben wurde ein Gemisch aus 10 Teilen Polyol A und 4,6 Teilen Polyisocyanat B.
Gemisch 4:
   Vorgelegt wurden 150 Teile Gipspulver, dazugegeben wurden zunächst 10 Teile Polyol B, dann 4,6 Teile Polyisocyanat B.
Gemisch 5:
   Vorgelegt wurden 100 Teile Gipspulver, dazugegeben wurden 6 Teile eines Gemisches aus 100 Teilen Polyol C und 1,2 Teilen Polyisocyanat B.
Gemisch 6:
   Vorgelegt wurden 172 Teile Gipspulver, dazugegeben wurden unter intensivem Mischen in einem Schaufelmischer erst 7,58 Teile Polyol A, danach 1,44 Teile Isocyanat C.

### Beispiel 1

A)
   Das 3 m lange Bindengewebe wurde auf eine streifenförmige und mit einem Trennmittel auf Silikonbasis besprühte Unterlage aus geglättetem Papier gelegt und unter einem Streuer so hindurchgefahren, daß in getrennten Ansätzen jeweils 1 m² des Baumwollgewebes mit jeweils 500 g frisch hergestellten Gemischen 1 bis 6 belegt wurde.
   Die jeweils aufgestreute Schicht wurde mit einer nichthaftend ausgerüsteten Rolle festgedrückt. Dann wurde das bestreute Bindengewebe durch den auf 0,07 mm Spaltbreite eingestellten Spalt eines Walzenstuhls mit polierten Edelstahlwalzen geführt. Hierbei wurde die Beschichtung auf dem Gewebe zu einer oberflächlich glänzenden festen opaken Schicht verdichtet, die fest auf dem Bindengewebe haftete. Dann wurde die Binde auf einen Dorn aus Polyolefin, der die Gestalt eines gelochten Rohres mit einem Durchmesser von 1 cm hatte, zu einem Wickel locker aufgerollt (Gesamtdurchmesser 6 cm). Hierbei wurde im Verlauf des Aufrollprozesses die verdichtete Schicht zwar in annähernd regelmäßigen Abständen senkrecht zur Längsachse gebrochen, haftete aber auch so an dem Bindengewebe.
B)
   Versuch A) wurde wiederholt, jedoch wurde vor dem Walzenstuhl auf das bestreute Bindengewebe ein zweites gleichartiges Gewebe aufgelegt, leicht angedrückt und dann der so gebildete Sandwich durch den Walzenstuhl gefahren. Es wurde wiederum eine glänzende, opake, fest haftende Materialschicht gebildet, die sich ohne abzuplatzen aufrollen ließ.
C)
   Der Versuch B) wurde wiederholt, jedoch wurde nun Gipspulver ohne Zusatz eines reaktiven Bindemittels eingesetzt. Nach dem Verdichten im Walzenstuhl wurde wiederum ein glänzender, nicht abrieselnder, jedoch weniger opaker Materialsandwich erhalten und aufgerollt.
D)
   Nicht erfindungsgemäß: Der Versuch B) wurde wiederholt, jedoch wurde der nach dem Aufbringen des zweiten Bindengewebes und leichtem Andrücken erhaltene lockere Sandwichverbund direkt, ohne Durchlauf durch den Walzenstuhl, aufgerollt.

Eine Prüfung des Gipsverlustes bei der trockenen Handhabung des gemäß den Versuchen A) bis D) hergestellten Bindenmaterials wurde wie folgt durchgeführt:
Die Wickel wurden in aufgerolltem Zustand 10 Tage bei 25°C gelagert, um die Abbindereaktion des reaktiven Bindemittels (soweit vorhanden) ablaufen zu lassen. Dann wurde von dem ca. 160 g wiegenden, 3 m Bindenmaterial enthaltendem Wickel auf einem ebenen Drahtsieb 1 m abgerollt und abgeschnitten. Das 1 m lange Bindenstück wurde dann von Hand auf dem Sieb um die lange Achse umgedreht, so daß die Oberseite nach unten zu liegen kam. Diese Prozedur wurde noch zweimal wiederholt, so daß letztlich die ursprüngliche Oberseite unten lag. Dann wurde das Bindenstück von Hand auf dem Sieb auf den ursprünglich verwendeten gelochten Wickeldorn von 1 cm Durchmesser wieder aufgerollt und vom Sieb genommen. Schließlich wurde der bei dieser Prozedur durch das Sieb gerieselte Gips ausgewogen. Je geringer die durchgerieselte Gipsmenge war, desto geringer sind die Gipsverluste bei der Handhabung der Binde, die noch nicht abgebundenen Gips enthält.

Diese Prüfung wurde jeweils dreimal durchgeführt und die ermittelten Gewichte des abgerieselten Gipsmaterials als Mittelwert in g/m bestimmt.

Folgende Werte wurden ermittelt:

| Binde aus Versuch | A | B | C | D (nicht erfindungsgemäß) |
|---|---|---|---|---|
| Bindemittelgemisch 1 | 4,5 | 0,9 | - | 21 |
| 2 | 7,0 | 2,1 | - | 26 |
| 3 | 4,9 | 1,3 | - | 22 |
| 4 | 4,7 | 1,3 | - | 23 |
| 5 | 5,1 | 2,0 | - | 24 |
| 6 | 4,0 | 0,9 | - | 21 |
| nur Gipspulver (ohne reaktives Bindemittel) | - | - | 3,4 | - |

Die erhaltenen Ergebnisse zeigen eine gute Trockenhandhabbarkeit der Binden, die mit der erfindungsgemäßen Verdichtung hergestellt werden, sogar, wenn kein reaktives Bindemittel für das Gipsmaterial verwendet wird. Die Ergebnisse zeigen auch den Verbesserungseffekt, der durch die Verwendung von zwei Gewebelagen erzielt wird.

Analoge Ergebnisse wurden erhalten, wenn anstelle des Baumwollgewebes ein gleichartiges Gewebe aus Hochmodul-Polyestergarn verwendet wurde.

### Beispiel 2

Mit den Binden hergestellt gemäß Beispiel 1 A), 1 B) und 1 D) unter Verwendung des Bindemittelgemisches 1 sowie mit Binden hergestellt nach Beispiel 1 C) wurden folgende Versuche ausgeführt:
Nach dem Austritt aus dem Walzenspalt wurde das mit der Bindemittelschicht versehene Baumwollgewebe auf 3 m abgelängt, auf einen rohrförmigen (1 cm Durchmesser) gelochten Wickeldorn aufgewickelt und dann auf einer Verpackungsanlage in Polyethylen-kaschierte Aluminiumfolie eingeschweißt, wie es bei der Gipsbindenherstellung nach konventioneller Methode geschieht, wenn der Wickel vor der Anwendung besonders geschützt werden soll.

Zur Applikation wurden die Wickel nach 2 Wochen aus der Verpackung genommen. Durch das nach dem Verdichtungsprozeß inzwischen erfolgte vollständige Abbinden des reaktiven Bindemittels (soweit vorhanden), waren die Wickel enthaltend die Binden aus den Beispielen 1 A), 1 B) und 1 D) ohne wesentliches Herausrieseln des Gipses handhabbar. Alle Wickel wurden 4 Sekunden lang (Typ D 15 Sekunden lang) in Wasser von 18°C senkrecht eingetaucht und manuell etwas gewalkt. Hierbei verlor lediglich der Wickel enthaltend die Binde aus Beispiel 1 C) eine merkliche Menge Gips (12 g). Dann wurde ein Zylinder aus Edelstahl, Durchmesser 8 cm, damit umwickelt und glattgestrichen. Nach 5 Minuten war der so hergestellte Gipsverband versteift. Das Härtungsverhalten und die Applizierbarkeit entsprachen bei den Binden aus den Beispielen 1 A), 1 B) und 1 D) mit dem Bindemittelgemisch 1 etwa einer auf übliche Weise hergestellten, mit 600 g Gipsmasse pro m² ausgerüsteten 10 cm-Gipsbinde, jedoch mit dem Unterschied, daß das Tränkwasser und die beim Walken herausgedrückte wäßrige Phase erheblich weniger den Charakter von Gipsbrühe, sondern mehr von leicht getrübtem Wasser hatte und daher ein wesentlich sauberes Arbeiten möglich wurde, als bei üblichen Gipsbinden.

Nach der Aushärtung wurde der so hergestellte rollenartig aufgewickelte Gipsverband von dem Zylinder abgestreift und bei 21°C bis zur Gewichtskonstanz getrocknet.

Dann wurde die 10 cm breite Rolle mit der Seite auf eine Waage aufgelegt und von oben mittig mit einem 6 cm²-Rundstempel zusammengedrückt. Die erzielbare maximale Tragfähigkeit des Wickels wurde dann als Maximumanzeige auf der Waage abgelesen. Sie ist ein Maß für die Festigkeit des Wickels.

Diese Versuche wurden an verschiedenen Wickeln des gleichen Typs fünfmal wiederholt und als Meßergebnisse der Mittelwert der Festigkeit aus den fünf Einzelmessungen erhalten, wie in der folgenden Tabelle angegeben:

| abgestreifter ausgehärteter Gipsverband mit einer Binde aus Beispiel | Festigkeit (kg) |
|---|---|
| 1 A) mit Bindemittelgemisch 1 | 63 |
| 1 A) mit Bindemittelgemisch 6 | 64 |
| 1 B) mit Bindemittelgemisch 1 | 68 |
| 1 C) ohne reaktives Bindemittel | 62 |
| 1 D) mit Bindemittelgemisch 1 | 65 |

Für eine auf übliche Weise hergestellte Gipsbinde (ohne Verdichtung und ohne reaktives Bindemittel) wurde auf entsprechende Weise eine Festigkeit von 59 kg ermittelt.

Das bedeutet, daß nach dem erfindungsgemäßen Verfahren die Festigkeiten der erhaltenen Wickel nach dem Abbinden in der gleichen Größenordnung oder besser liegen, als bei konventionellen Gipsbinden. Die Ergebnisse zeigen auch, daß der Sandwichaufbau zu einer Verbesserung der Festigkeit führt, was in Verbindung mit der lösungsmittelfreien und technisch einfachen Herstellweise, der guten Trockenhandhabbarkeit und der geringen Gipsverluste beim Tränken, einen bedeutenden technischen und ökologischen Fortschritt bedeutet.

### Beispiel 3

Es wurde wie in Beispiel 1 A) mit dem Bindemittelgemisch 1 und wie in Beispiel 1 C) verfahren. Es wurde jedoch eine Walze des Walzenstuhls durch eine achsparallel geriffelte Walze mit Stegbreiten von 3,4 mm, Zwischenraumbreiten von 1,5 mm und Zwischenraumtiefen von 2,5 mm ersetzt. Die Walzen wurden auf eine Spaltbreite von 0,085 mm zwischen Riffelwalze und glatter Walze eingestellt. Das Bindengewebe wurde mit 800 g/m² Bindemittel gleichmäßig bestreut.

Durch den nunmehr intermittierenden Verdichtungsprozeß wurde das Bindemittel auf dem Bindengewebe streifenförmig, quer zur Gewebelänge auf dem Gewebe fixiert. Bei dem an den Walzenstuhl anschließenden Transport der Binde zu einer Abläng- und Wickelvorrichtung wurde durch schwaches Rütteln die nicht durch die Riffeln der Walze verdichtete Menge Bindemittel durch die Gewebemaschen abgerieselt und dem Beschichtungsprozeß wieder zugeführt. Die Verdichtungsbezirke haften auf den Gewebebahnen und wurden in den Wickel eingebracht.

Bei dieser Anordnung der Beschichtung ist eine besonders gute Aufwickelbarkeit und Trockenhandhabbarkeit gegeben. Beim Tauchen in Wasser reichten bei senkrechtem Eintauchen des Wickels Eintauchzeiten von ca. 2 Sek., um eine vollständige Durchfeuchtung des Wickels zu erzielen. Das Applikationsverhalten und die Endfestigkeiten entsprachen den Produkten aus den Beispielen 1A) und 1C).

Analoge Ergebnisse wurden erhalten, wenn auf das Gewebe eine Abmischung gleicher Gewichtsteile Portlandzement und Bindemittelgemisch aufgestreut wurde.

### Beispiel 4

Ein 2 Wochen abgelagerter 3 m-Wickel erhalten gemäß 1 B) mit dem Bindemittelgemisch 1 wurde trocken abgerollt und zu einem Stapel von 20 cm Länge aufgefaltet. Diese Abrollen und zu einem Stapel auflegen führte zu geringen Gipsverlusten durch Abrieseln von weniger als 4,5 g. Dann wurde der Stapel unter Erhalt seiner Geometrie 3 Sek. in Wasser getaucht, durch Senkrechthalten abgetropft, wobei nahezu kein Gipsleim ablief und dann als Longuette in einen auf ein Model gewickelten Handversteifungsverband (Finger-Handgelenksbereich) eingearbeitet. Hier erfolgte die Erhärtung in Analogie zu konventionellen Gipsverbandsmaterialien im Verlaufe von 3 bis 6 Minuten.

### Beispiel 5

Auf die Klebstoffseite eines 4 m langen und 12 cm breiten Textilklebebandes wurden parallel im Abstand von 2 mm gespritzte Streifen aus Polycarbonat von 12 cm Länge, 3 mm Breite und 2 mm Dicke aufgelegt und festgepreßt, so daß eine Art Panzerkette oder Strickleiterstruktur-Band entstand. Dieses Band wurde unter das Bindengewebe gelegt und dann mit 600 g/m² einer Gips-Bindemittel-Mischung bestreut, so daß über die Fläche des Gewebes eine gleichmäßige Bestreuung erfolgte. Das Gips-Bindemittel-Gemisch war durch intensives Mischen von 200 Teilen Halbhydrat (Gipspulver) zunächst mit 7,65 Teilen des Polyols C erhalten worden.

Anschließend wurde das bestreute Gewebe mit der darunterliegenden "Panzerkette" durch ein Paar übereinander angeordnete glatte Edelstahlwalzen (Spaltbreite 2,07 mm) gefahren. Über eine Umlenkrolle, an der der nicht von den Polycarbonatelementen der "Panzerkette" verdichtete Anteil des aufgestreuten Pulvers abrieselte und in den Streuer zurückgeführt wurde, wurde das nunmehr eine Art Strickleiter-Struktur aufweisende Bindenmaterial lose zu einem 6 cm Durchmesser aufweisenden Wickel aufgerollt. Anschließend wurde der auf einem gelochten, rohrförmigen Kunststoffwickeldorn von 1 cm Durchmesser befindliche Wickel in eine dicht schließende Polyethylendose verpackt.

Nach 7 Tagen wurde der Wickel entnommen und durch Eintauchen in Wasser (19°C, Eintauchzeit 3 Sekunden, senkrechte Eintauchrichtung) vollständig benetzt. Das ablaufende Wasser war nur leicht getrübt und hatte nicht den Charakter von Gipsbrühe. Die Gipsbinde konnte sehr sauber abgewickelt und wie in Beispiel 2 beschrieben in einen Test-Gipsverband überführt werden. Der Gipsverlust bei Überprüfung der Trockenhandhabbarkeit entsprechend Beispiel 1 lag bei 1,5 g/m und die Festigkeit des entsprechend Beispiel 2 erhaltenen Test-Gipsverbandes bei 64 kg, also im normalen Rahmen. Aufgrund der Stickleiterstruktur konnte die Binde dieses Typs ähnlich wie die gemäß Beispiel 4 erhaltene Binde, jedoch ohne Brechen der Gipsauflage und daher mit noch geringerem Gipsverlust zu Longetten aufgerollt und verarbeitet werden.

### Beispiel 6

Beispiel 5 wurde wiederholt, jedoch wurde zum Bestreuen ein Gips-Bindemittel-Gemisch eingesetzt, das durch intensives Mischen von 170 Teilen Gipspulver mit 7,58 Teilen Polyol A und 1,45 Teilen Isocyanat C erhalten worden war. Die anwendungstechnischen Eigenschaften waren ähnlich wie im Beispiel 5.

## Patentansprüche

1. Verfahren zur Herstellung von hydraulische Bindemittel enthaltenden Versteifungsmaterialien, dadurch gekennzeichnet, daß man in Abwesenheit von Blähtonkugeln
- ein hydraulisches Bindemittel in Pulverform auf ein flächiges Material aufbringt,
- das hydraulische Bindemittel im Kontakt mit dem flächigen Material verdichtet und
- das so mit verdichtetem hydraulischem Bindemittel versehene flächige Material zu Rollen aufwickelt.

2. Verfahren nach Anspruch 1, dadurch gekennzeichnet, daß man die Verdichtung des hydraulischen Bindemittels auf einer oder zwischen 2 oder mehr Lagen flächigen Materials durchführt.

3. Verfahren nach Ansprüchen 1 und 2, dadurch gekennzeichnet, daß das hydraulische Bindemittel auf dem flächigen Material in Form von einzelnen Querstreifen einseitig oder beidseitig angeordnet wird,

4. Verfahren nach Ansprüchen 1 bis 3, dadurch gekennzeichnet, daß man zusätzlich zu dem hydraulischen Bindemittel reaktive Bindemittel beisetzt.

5. Verfahren nach Ansprüchen 1 bis 4, dadurch gekennzeichnet, daß man medizinisch anwendbare Gipsbinden herstellt, indem man
- ein hydraulisches Bindemittel auf Gipsbasis mit einem reaktiven Bindemittel vermischt,
- diese Mischung auf ein flächiges Material aufbringt,
- die so erhaltene Beschichtung gegebenenfalls mit einem weiteren flächigen Material abdeckt,
- das Bindemittelgemisch bis zum Verlust seiner Rieselfähigkeit verdichtet,
- das so mit einer verdichteten Bindemittelschicht versehene Produkt locker zu Rollen aufwickelt und
- vor, während und/oder nach dem Aufwickeln die Abbindereaktion des reaktiven Bindemittels ablaufen läßt.

6. Verfahren nach Ansprüchen 1 bis 5, dadurch gekennzeichnet, daß als hydraulische Bindemittel teilhydratisierte Gipse, α-Gips, β-Gips, Anhydrit, Portlandzement, Tonerdeschmelzzement, Aluminiumsilikat-Schnellzement, Sorellzement, Zinkoxidzement und/oder Puzzolanzement und als reaktive Bindemittel Silikate vom Wasserglastyp, Epoxide, Polyepoxide, Epoxid-Härter-Kombinationen, Cyanacrylate, Acrylate, Vinylester, Allylether, Silane, Siloxane, Silikone, Alkydharze, reaktive Alkydharzverdünner, Cyanatharze, Phenolharze, Formaldehydharze, Methylolverbindungen, Methylolether und/oder Isocyanate, die unter Bildung von Polyurethanen, Polyharnstoffen, Polycarbodiimiden und/oder Polyisocyanuraten zur Reaktion gebracht werden können, vorzugsweise Kombinationen von Polyisocyanaten mit Polyolen, zum Einsatz gelangen.

7. Verfahren nach Ansprüchen 1 bis 6, dadurch gekennzeichnet, daß man auf 1 m² des flächigen Materials 100 bis 1 000 g hydraulische Bindemittel oder Gemische aus hydraulischen und reaktiven Bindemitteln aufbringt, wobei, bezogen auf hydraulische Bindemittel, 0 bis 50 Gew.-% reaktive Bindemittel verwendet werden.

8. Verfahren nach Ansprüchen 1 bis 7, dadurch gekennzeichnet, daß man die Verdichtung mittels eines Durchlaufs durch ein oder mehrere Walzenpaare vornimmt.

9. Verfahren nach Ansprüchen 1 bis 8, dadurch gekennzeichnet, daß man die Verdichtung in Form von Streifen oder sonstigen Mustern zwischen Walzen vornimmt.

10. Verwendung von Versteifungsmaterialien, hergestellt nach den Ansprüchen 1 bis 9 im medizinisch/orthopädischen Bereich.

## Claims

1. Process for the production of stiffening materials containing hydraulic binders, characterised in that in the absence of expanded clay balls
- a hydraulic binder in powder form is applied to a sheetlike material,
- the hydraulic binder is compacted in contact with the sheetlike material and
- the sheetlike material thus provided with compacted hydraulic binder is wound into rolls.

2. Process according to Claim 1, characterised in that compaction of the hydraulic binder is carried out on one or between two or more layers of sheetlike material.

3. Process according to Claims 1 and 2, characterised in that the hydraulic binder is arranged on the sheetlike material in the form of individual horizontal stripes on one side or both sides.

4. Process according to Claims 1 to 3, characterised in that, in addition to the hydraulic binder, reactive binders are added.

5. Process according to Claims 1 to 4, characterised in that plaster bandages usable in medicine are produced by
- mixing a plaster-based hydraulic binder with a reactive binder,
- applying this mixture to a sheetlike material,
- if desired covering the coating thus obtained with a further sheetlike material,
- compacting the binder mixture until it loses its flowability,
- winding the product thus provided with a compacted binder layer loosely into rolls and
- allowing the setting reaction of the reactive binder to proceed before, during and/or after winding.

6. Process according to Claims 1 to 5, characterised in that the hydraulic binders used are partially hydrated gypsums, α-gypsum, β-gypsum, anhydrite, Portland cement, high-alumina cement, aluminosilicate quick-setting cement, Sorell cement, zinc oxide cement and/or Pozzolan cement and the reactive binders used are silicates of the waterglass type, epoxides, polyepoxides, epoxy curing agent combinations, cyanoacrylates, acrylates, vinyl esters, allyl ethers, silanes, siloxanes, silicones, alkyd resins, reactive alkyd resin thinners, cyanate resins, phenolic resins, formaldehyde resins, methylol compounds, methylol ethers and/or isocyanates which can be made to react with the formation of polyurethanes, polyureas, polycarbodiimides and/or polyisocyanurates, preferably combinations of polyisocyanates with polyols.

7. Process according to Claims 1 to 6, characterised in that 100 to 1,000 g of hydraulic binder or mixtures of hydraulic and reactive binders are applied to 1 m² of the sheetlike material, 0 to 50% by weight of reactive binders, relative to hydraulic binder, being used.

8. Process according to Claims 1 to 7, characterised in that compaction is carried out by means of a passage through one or more pair(s) of rolls.

9. Process according to Claims 1 to 8, characterised in that compaction is carried out in the form of stripes or other patterns between rolls.

10. Use of stiffening materials produced according to Claims 1 to 9 in the medical/orthopaedic sector.

## Revendications

1. Procédé pour la préparation de matériaux de raidissement contenant des liants hydrauliques, caractérisé qu'en l'absence de sphères d'argile expansée
- on applique un liant hydraulique dans la forme de poudre sur un matériau en nappe,
- on compacte le liant hydraulique au contact avec le matériau en nappe et
- on enroule en rouleaux le matériau en nappe muni ainsi du liant hydraulique compacté.

2. Procédé selon la revendication 1, caractérisé en ce qu'on réalise le compactage du liant hydraulique sur une ou entre deux ou plusieurs couches du matériau en nappe.

3. Procédé selon les revendications 1 ou 2, caractérisé en ce que le liant hydraulique est disposé sur le matériau en nappe sur une face ou sur les deux faces dans la forme de rayures perpendiculaires individuelles.

4. Procédé selon les revendications 1 à 3, caractérisé en ce qu'on dispose en outre auprès du liant hydraulique un liant réactif.

5. Procédé selon les revendications 1 à 4, caractérisé en ce qu'on prépare des bandes de plâtre pouvant être appliquées dans le domaine de la médecine, en ce que
- on mélange un liant hydraulique à base de plâtre avec un liant réactif,
- on applique ce mélange sur un matériau en nappe,
- on recouvre le revêtement ainsi obtenu éventuellement avec un autre matériau en nappe,
- on compacte le mélange de liants jusqu'à la perte de sa faculté d'écoulement,
- on enroule en rouleaux de manière lâche le produit ainsi muni d'une couche de liants compactée et
- on laisse se faire la réaction de prise du liant réactif avant, pendant et/ou après l'enroulement.

6. Procédé selon les revendications 1 à 5, caractérisé en ce qu'on utilise en tant que liants hydrauliques des plâtres partiellement hydratés, du plâtre α, du plâtre β, de l'anhydrite, du ciment de Portland, du ciment alumineux fondu, du ciment rapide de silicate d'aluminium, du ciment à la magnésie, du ciment d'oxyde de zinc et/ou du ciment pouzzolanique et en tant que liants réactifs des silicates du type verre soluble, des époxydes, des polyépoxydes, des combinaisons époxydes-durcisseurs, des cyanacrylates, des acrylates, des esters vinyliques, des éthers allyliques, des silanes, des siloxanes, des silicones, des résines alkydes, des diluants réactifs de résines alkydes, des résines cyanates, des résines phénols, des résines formaldéhydes, des composés du méthylol, du méthyloléther et/ou des isocyanates qu'on fait réagir pour former des polyuréthanes, des polyurées, des polycarbodiimides et/ou des polyisocyanurates, de préférence des combinaisons de polyisocyanates avec des polyols.

7. Procédé selon les revendications 1 à 6, caractérisé en ce qu'on applique sur 1 m² du matériau en nappe 100 à 1000 g de liants hydrauliques ou de mélanges de liants hydrauliques réactifs, de 0 à 50 % en poids, rapportés aux liants hydrauliques de liants réactifs étant utilisés.

8. Procédé selon les revendications 1 à 7, caractérisé en ce qu'on réalise le compactage au moyen d'un passage à travers une ou plusieurs paires de cylindres.

9. Procédé selon les revendications 1 à 8, caractérisé en ce qu'on réalise le compactage dans la forme de rayures ou d'autres motifs entre des cylindres.

10. Utilisation de matériaux de raidissement préparés selon les revendications 1 à 9 dans le domaine médical/orthopédique.
